# EUROPEAN PATENT APPLICATION

(11) **EP 3 786 507 A1**
(43) Date of publication of application: **03.03.2021**
(21) Application number: 19194987.4
(22) Date of filing: 02.09.2019
(51) Int. Cl.: F16L 55/26, G01M 3/24, G01D 21/00, G01D 11/30, A61B 5/00, E21B 47/01

(54) **CAPTURED IN-PIPE SENSOR**

(71) Applicant: ABB Schweiz AG, 5400 Baden (CH)
(72) Inventor: KEEPING, Sean, Shortlands, BR2 0HP (GB); SZÁSZ, Paul, 68723 Plankstadt (DE); LINCOLN, David, St Neots, PE191LN (GB); YEATMAN, Eric, London, SW7 2AZ (GB); BOYLE, David, London, SW7 2AZ (GB); HOLMES, Andrew, London, SW7 2AZ (GB); WILLIAMS, Daryl, London, SW7 2AZ (GB)
(74) Representative: Maiwald Patent- und Rechtsanwaltsgesellschaft mbH

(57) **Abstract**

The invention relates to a sensor system (10) and a method for sensing at least one process parameter and for generating a sensed value representing the at least one process parameter inside a passage (70), for instance in a pipe. The sensor system (10) is configured for sensing at least one process parameter and for generating a sensed value representing the at least one process parameter at a predefined location (75) inside the passage (70), which comprises a medium (72). The sensor system (10) comprises a sensor (50), configured to measure the at least one process parameter and to generate a sensed value representing the at least one process parameter; a movement mechanism (40), configured to move the sensor system (10) inside the passage (70) to the predefined location (75); a holding mechanism (30), configured to hold the sensor system (10) at the predefined location (75), after the sensor system (10) has moved to the predefined location (75); and a communication mechanism (60), configured to enable data communication to and from the sensor system (10), data communication being related to aspects including at least one of controlling the movement mechanism (40), the holding mechanism (30), and/or the sensor (50).

## Description

### FIELD OF THE INVENTION

The invention relates to a sensor system and a method for conducting a measurement inside a passage, for instance in a pipe. Furthermore, the invention relates to a use of a measuring device.

### BACKGROUND OF THE INVENTION

In many cases, measuring process parameters inside a passage may require invasive installation of sensors at each location, where process parameters are to be measured. For example, if a tube with a closed section is to be measured, there may be a requirement to make an opening in the tube, through which the sensor can be inserted. This may have drawbacks, e.g. it may raise additional cost and may have an impact on the tube's integrity.

### DESCRIPTION OF THE INVENTION

It is therefore an objective of the invention to conduct a measurement inside a passage without making an opening into the passage.

This objective is achieved by the subject-matter of the independent claims. Further embodiments are evident from the dependent patent claims and the following description.

One aspect relates to a sensor system for sensing at least one process parameter and for generating a sensed value representing the at least one process parameter at a predefined location inside a passage comprising a medium. The sensor system comprises a sensor, configured to measure the at least one process parameter and to generate a sensed value representing the at least one process parameter, a movement mechanism, configured to move the sensor inside the passage to the predefined location, and a holding mechanism, configured to hold the sensor at the predefined location, after the sensor has moved to the predefined location. Furthermore, the sensor system comprises a communication mechanism, configured to enable data communication to and from the sensor system, wherein the data communication is related to aspects, which include controlling at least one of the movement mechanism, the holding mechanism, and/or the sensor.

The passage may be essentially closed, it may be partly open and/or in sections open. Examples of a passage are a channeling, a tube, a pipeline for conducting (e.g.) oil or gas, a blood vessel or vasculature and/or other types of passages. An inner wall of inner surface of the passage may be essentially smooth. The passage may be filled, at least partly, with a medium. The medium may be floating inside the passage and/or may stand still. The medium may be, e.g., a fluid, i.e. a liquid or a gas, a bulk, and/or a mixture of these, e.g. a suspension. The predefined location may be one or more locations. The predefined location may be sited at a border, at a mid of the passage, and/or anywhere in between.

The movement mechanism is configured to move the sensor system inside the passage. The design of the movement mechanism may depend on the floating-characteristic of the medium, i.e. if the medium usually floats continuously or in an intermittent way, or if it stands still. In embodiments of the sensor system, where the sensor system is to be used for an un-moved medium, the movement mechanism may comprise a propagation component. The propagation component may comprise a device-internal drive, such as a turbine or another motor, and/or a device-external drive, such as an external magnetic field that drives a ferromagnetic piece (or a magnet etc.) in the measuring device. In embodiments that are configured to use the sensor system in a continuously floating medium, a steering mechanism may be sufficient as the movement mechanism. In embodiments that are configured to use the sensor system in a passage without branches and having a continuously floating medium, the movement mechanism may be a minimal one, e.g. just having an influence on the form of the sensor system, e.g. having the form of a sphere, an ellipsoid, an egg, or the like.

The holding mechanism is configured to hold the sensor system at the predefined location, after the sensor system has moved to the predefined location. Having arrived at the predefined location, the sensor system is captured at this point, where it then stays and makes measurements continuously, periodically, or when requested. The holding can be done by any means. The holding mechanism may depend on the site of the predefined location, where it is supposed to conduct the measuring. For instance, if the sensor system is to conduct the measuring close to the wall, e.g. a magnetic holding mechanism may be used, for conducting the measuring in the mid of the passage, a spider-like mechanism may be used. The holding may include a slight drifting, particularly in a fast flowing medium.

The sensor system comprises a sensor, which is configured to measure the at least one process parameter and to generate a sensed value representing the at least one process parameter. The sensor system may comprise one sensor or a plurality of sensors. The kind of sensor(s) may depend on the kind of measurement to be conducted. For instance, the sensor may be an optical one (e.g., a brightness sensor, or a camera), an acoustic sensor, a sensor for magnetic and/or electric fields, a pressure sensor, a temperature sensor, a pH sensor, a sensor for the velocity of the medium, and/or another sensor, including other types of chemical sensors and/or microstructured sensors, e.g. so-called MEMS-based sensors (MEMS: Micro-Electro-Mechanical Systems).

The sensor system comprises a communication mechanism, configured to control the movement mechanism, the holding mechanism, and/or the sensor. The communication mechanism may use electro-magnetic means (e.g. radio), magnetic means (e.g. transponder), acoustic means (e.g. a kind of loudspeaker), and/or optical means (e.g. an LED). Controlling the movement mechanism may comprise to determine the direction and/or the velocity of the measuring device. Controlling the holding mechanism may comprise to activate and/or to release the sensor system and/or a part of it. Controlling the sensor may comprise to receive and/or to exchange sensor data, and/or to change parameters of the sensor, e.g. its measuring ranges and/or its measurement area.

Thus, the sensor system is able to conduct a measurement inside a passage without making an opening into the passage. In addition, controlling the movement and the holding makes the sensor system highly versatile for a broad range of measurements, within various types of passages. This allows a measurement of process parameters at multiple locations, e.g. in a piping system or another kind of passage, without invasive installation of sensors at any location. Thus, data (and other) communication to and from the sensor system may be achieved without piercing - or otherwise essentially changing - the passage and/or the surface of the passage at the capture location.

In various embodiments, the sensor system further comprising an energy supply mechanism, wherein the energy supply mechanism comprises an energy storage and/or an energy generator. The energy storage may be an accumulator and/or a capacitor, e.g. an electrolytic capacitor, and/or a long-life battery. The energy generator may be configured to generate energy based on an electro-magnetic, magnetic, acoustic, optical and/or thermal source or environment. The energy may be sent from outside, e.g. by a magnetic field (and/or other means) that is arranged to load the energy storage of the measuring device. The energy generator may use so-called energy harvesting, from flow (e.g. using a turbine), in-flow vibration or eddies, or ion flow (e.g. by Hall effect). Power transmission and data transmission may be combined into a single system with common transducers. An example may be an RFID-system (RFID: radio-frequency identification). The energy supply mechanism may be a basis for an - at least partly - independence of the measuring device. One of these aspects may be to save sensor data, e.g. temporarily for periods of disturbed transmittance. Another aspect may be to control the movement mechanism and/or the holding mechanism in an at least partly autonomous way.

In some embodiments, the energy generator comprises a magnetic and/or electro-magnetic coupler and/or energy harvesting means. An example of a magnetic coupler may be a transponder, an RFID-chip, or the like. At least some of these embodiments allow to combine energy transmission and communication, e.g. of data and/or control commands.

In various embodiments, the movement mechanism comprises a propulsion mechanism, a broadened portion, a rudder, and/or a sail. The propulsion mechanism may comprise a propeller, an impeller, a turbine, a pump-jet or water jet, and/or other kind of motors. The broadened portion may comprise a mechanism that broadens (and/or un-broadens) a part of the sensor system, in order to increase (or decrease, respectively) the area by means of which the floating medium pushes the sensor system, thus changing its drag coefficient. The rudder or rudder blade may be used for steering the sensor system in the medium. The sail, or something that is configured to produce an under-pressure on one side, may be used for steering the sensor system in the floating medium.

In various embodiments, the holding mechanism comprises magnetic means, friction means, and/or Van der Waals means. Magnetic means may comprise an electromagnetic mechanism in the sensor system, which is configured to fix the device on a pipe with a ferromagnetic surface and/or material. Magnetic means may comprise a ferromagnetic piece in the sensor system, which is configured to be fixed by an external module that is configured to exert magnetic force on the ferromagnetic piece. These are reliable, easy-to-handle and inexpensive means to realize the holding. Friction means may comprise a vacuum cup, e.g. combined with a pump configured to suck the vacuum cup. This is particularly advantageous for passages with at least partly smooth surfaces. Friction means may comprise an element with high friction that is configured to be pressed against the surface of the passage. By this, measurements can be conducted, e.g., in a middle area of the passage. Van der Waals means may comprise a ribbon or the like having high Van der Waals adhesion (and/or possibly otherwise adhesive means). This may be an energy-saving means and/or it may be particularly gentle to the passage's surface.

In various embodiments, the communication mechanism comprises a transceiver, which is configured to communicate electromagnetically, magnetically, optically, and/or acoustically. In some embodiments, the communication mechanism may be combined with a energy-transmission mechanism.

In an embodiment, the movement mechanism and/or the holding mechanism comprises: a body, a leg, which is mounted on the body in a moveable way, and a foot, which is mounted on the leg. There may be one leg or a plurality of legs. This embodiment may look "spider-like". The moving capabilities of the legs at least comprise a spreading and a folding w.r.t. a middle axis of the measuring device. The foot, which is mounted on the leg, may be designed as an end-portion, which is similar to the others parts of the leg. The foot may be broadened, e.g. for enhancing the frictional effect. The foot may be moveable, relative to the leg, e.g. by a spring or a motor. The foot may comprise ferromagnetic, electromagnetic, and/or magnetic elements for magnetic-based holding. The foot may, additionally or as an alternative, comprise vacuum and/or Van der Waals elements. For holding the sensor system, the magnetic, etc., means may be used, respectively. For holding, the legs may be spread, until they (or the one leg) reach the surface of the passage. For an accelerated movement in a floating medium, the legs may be spread (at least not all of the legs reaching the surface of the passage). For a decelerated movement in a floating medium, the legs may be folded; this may, additionally, lead to turbulences, thus decelerating the measuring device.

In an embodiment, spreading the leg comprises means for axially turning the leg, thus increasing the drag coefficient of the leg. For this, the leg may have a flat and a broad side, e.g. implemented as a rectangle or an ellipsoid. For an accelerated movement in a floating medium, the legs may be spread and additionally turned, thus bringing the thicker side into the floating direction of the floating medium, for getting a higher drag coefficient (aka c_{w} or cₓ value). For a non-floating medium, the higher drag coefficient may be used for increasing the deceleration of the movement. In addition, the leg or the legs may have a concave portion. This may save weight and may further increase the drag coefficient.

In various embodiments, the passage, where the sensor system is to conduct the measurement, is partly and/or in sections open, and/or is designed as a container, a tube, a pipeline, and/or as a vasculature. The design of the sensor system for various types of passages may enhance the versatility of the measuring device. The physical dimensions of the sensor system may range - dependent on the application - from smaller than one millimetre to several centimetres.

A further aspect relates to a method for sensing at least one process parameter and for generating a sensed value representing the at least one process parameter, by a sensor system, at a predefined location inside a passage comprising a medium. The method comprises the steps of:
inserting the sensor system into the medium of the passage;
guiding the sensor system, by means of a movement mechanism, to the predefined location;
holding the sensor system, by means of a holding mechanism, at the predefined location; and
measuring the at least one process parameter and generate a sensed value representing the at least one process parameter, by means of a sensor, at the predefined location.

The inserting may be done manually and/or by a tool, including a robotic. The sensor system may be injected into the medium at one opening.

The movement mechanism may be designed as pointed out above and/or below. For instance, the guiding to the predefined location may be achieved by being propagating the sensor system in the flow, by a propulsion mechanism built into the sensor system, or by a propulsion system operated from outside the pipe and coupled to the module, e.g., in a contactless way (e.g. by magnetic means).

One option or embodiment for implementing the holding mechanism, for capturing the module or the sensor system, is for the module structure to make contact with the pipe inside a wall or surface of the passage with "feet", while presenting minimal surface area to the flow so as not to impede it once captured. This may be called a "spider" structure. Capture could then be achieved by applying a magnetic force to the foot through the pipe at the capture location.

The measurement is conducted by means of a sensor, after having reached the predefined location. The measurement may be conducted continuously, periodically, or when requested.

In various embodiments, the method comprises the further step of releasing the sensor system, by means of the holding mechanism. The releasing may be achieved, e.g. by releasing the magnetic and/or the frictional force. In an embodiment, after capture of one foot, actuators in the remaining legs could be used to release these from the pipe walls or to fold the leg towards a body of the measuring device.

In various embodiments, holding the sensor system comprises building a detachable connection between the sensor system and the passage, and/or keeping the structure of the passage essentially unchanged. Thus, no drilling, no anchor, nor other fixing-mechanisms are required. Thus, there is no "violating" of the surface of the passage (or only a minimal one, like scratching). This contributes to a high durability of the passage.

In various embodiments, at least one of inserting, guiding, and/or holding the sensor system, measuring the at least one process parameter and generating a sensed value representing the at least one process parameter, and/or releasing the sensor system is controlled by a communication mechanism. The communication mechanism may be implemented by using wireless transfer, e.g. via magnetic coupling (e.g. Near Field Communication, NFC, and/or RFID), or electromagnetic coupling (e.g. Bluetooth), and/or other wireless communication mechanisms.

In some embodiments, guiding the sensor system comprises to move the sensor system, by means of a propulsion mechanism. And/or, wherein the medium is a flowing medium and wherein the guiding comprises a steering, by means of a rudder and/or a sail.

A further aspect relates to a use of a sensor system as described above and/or below or a method as described above and/or below for measuring at least one process parameter and generating a sensed value representing the at least one process parameter inside a passage at a predefined location.

A further aspect relates to a computer program product including computer program code for controlling one or more processors, which are configured to perform a method as described above and/or below for conducting a measurement inside a passage at a predefined location.

A further aspect relates to a computer-readable storage medium where a computer program or a computer program product as described above is stored on.

### BRIEF DESCRIPTION OF THE DRAWINGS

The subject matter of the invention will be explained in more detail in the following text with reference to preferred exemplary embodiments, which are illustrated in the attached drawings, in which:
**Fig. 1** schematically shows a sensor system according to an embodiment;
**Fig. 2** schematically shows a sensor system according to a further embodiment;
**Fig. 3** shows a method for measuring at least one process parameter according to an embodiment.

The reference symbols used in the drawings, and their meanings, are listed in summary form in the list of reference symbols. In principle, identical parts are provided with the same reference symbols in the figures.

### DETAILED DESCRIPTION OF PREFERRED EMBODIMENTS

The reference symbols used in the drawings, and their meanings, are listed in summary form in the list of reference symbols. In principle, identical or similar parts are provided with the same reference symbols in the figures.

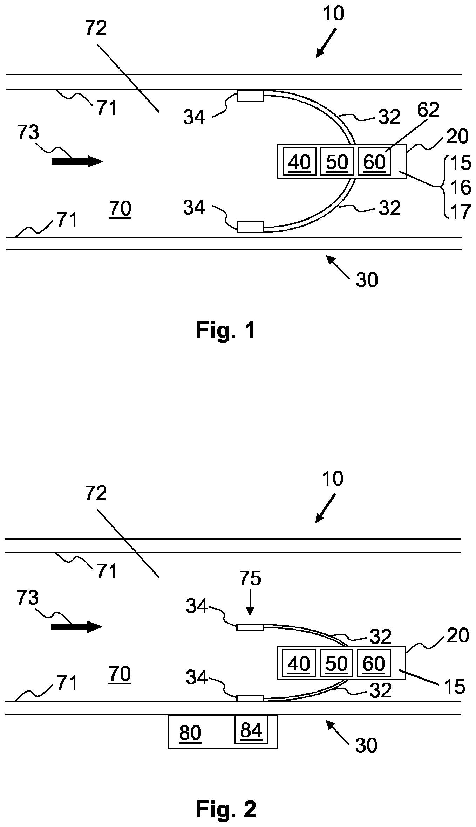

**Fig. 1** schematically shows a sensor system 10 according to an embodiment. The sensor system 10 is sited inside a passage 70. The passage may be essentially closed, it may be partly open and/or in sections open. The passage 70 comprises a medium 72. The medium 72 may be, e.g., a fluid, i.e. a liquid or a gas, a bulk, and/or a mixture of these, e.g. a suspension. In the scenario shown, the medium 72 floats into the direction of arrow 73. A sensor system 10 is located inside the passage 70, but without touching the walls 71 of the passage 70.

The sensor system 10 comprises a body 20. Within the body 20 and/or attached to it, a movement mechanism 40, a holding mechanism 30, a sensor 50, and a communication mechanism 60 is arranged. The communication mechanism 60 comprises a transceiver 62, which is configured to communicate electromagnetically, magnetically, optically, and/or acoustically. Furthermore, a power supply mechanism 15 is arranged in the body 20. The power supply mechanism 15 comprises an energy storage 16 and an energy generator 17. The holding mechanism 30 comprises legs 32 and feet 34. The feet 34 are arranged or mounted at a first end-portion of the legs 32. A second end-portion of the legs 32 is mounted on the body 20 in a moveable way. The movement mechanism 40 may comprise a motor or turbine arranged at or in the body 20 and/or a rudder, a sail, and/or the like for steering the sensor system 10. The movement mechanism 40 further comprises means for spreading the legs 32, thus accelerating the sensor system 10 in the floating medium 72 (however, essentially without touching the walls 71 of the passage 70). By these means of the movement mechanism 40, the sensor system 10 is driven towards a predefined location 75 (see **Fig. 2**). The driving is controlled by the communication mechanism 60, possibly by using a control unit. Such a control unit may be part of the communication mechanism 60 or arranged separately inside or near the body 20.

**Fig. 2** schematically shows a sensor system 10 according to a further embodiment. This embodiment may be similar to the embodiment shown in **Fig. 1**. Similar or identical parts are provided with the same reference symbols. **Fig. 2** schematically shows a scenario, in which the sensor system 10 has reached a predefined location 75. In this embodiment, at least the feet 34 carry ferromagnetic elements. At the predefined location 75, an external module 80 is arranged. The external module 80 carries an electromagnetic element 84. The external module 80 may further carry a device to communicate with the communication mechanism 60 and/or the transceiver 62. The communication mechanism 60 may be configured to enable data communication to and from the sensor system 10, e.g. by means of the communication mechanism 60 and/or the transceiver 62. The data communication may be related to aspects, which include controlling at least one the movement mechanism 40, the holding mechanism 30, and/or the sensor 50.

When activated, the electromagnetic element 84 fixes the ferromagnetic elements of the at least one foot 34 at a wall 71 of the passage 70 in a detachable way. Furthermore, to reduce the drag coefficient of the sensor system 10, the legs 32 are folded. In this predefined location 75 inside the passage 70, measurements may be conducted by the sensor 50. The measurement values may be transferred to the external module 80, e.g. by wireless communication means. The measurement values may be stored in a memory inside sensor system 10, either temporarily or until a series of measurements has been conducted. In the latter case, measurement values may be read from the memory after the sensor system 10 has left the passage. The measurements may comprise sensing at least one process parameter and generating a sensed value representing the at least one process parameter.

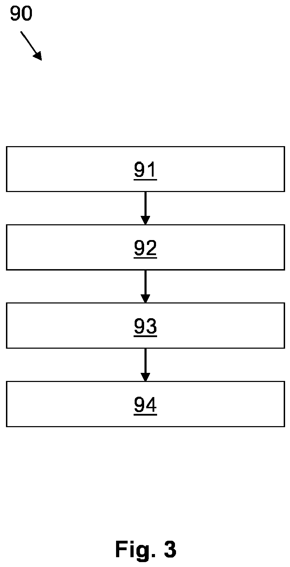

**Fig. 3** shows a flow diagram 90 of a method for sensing at least one process parameter and for generating a sensed value representing the at least one process parameter according to an embodiment. The measurement is conducted by a sensor system 10 (cf. **Fig. 1** or **2**), at a predefined location 75 inside a passage 70, which comprises a medium 72. In a step 91, the sensor system 10 is inserted into the medium 72 of the passage 70. In a step 92, the sensor system 10 is guided, by means of a movement mechanism 40, to the predefined location 75. In a step 93, the sensor system 10 is held, by means of a holding mechanism 30, at the predefined location 75. In a step 94, the sensor system 10 measures the at least one process parameter and generates a sensed value representing the at least one process parameter, by means of a sensor 50, at the predefined location 75.

### LIST OF REFERENCE SYMBOLS

- 10: sensor system
- 15: energy supply mechanism
- 16: energy storage
- 17: energy generator
- 20: body
- 30: holding mechanism
- 32: leg(s)
- 34: foot, feet
- 40: movement mechanism
- 50: sensor
- 60: communication mechanism
- 62: transceiver
- 70: passage
- 71: passage wall
- 72: medium
- 73: arrow
- 75: predefined location
- 80: external module
- 84: electromagnetic element
- 90: flow diagram
- 91 - 94: steps

## Claims

1. A sensor system (10) configured for sensing at least one process parameter and for generating a sensed value representing the at least one process parameter at a predefined location (75) inside a passage (70) comprising a medium (72), the sensor system (10) comprising:
a sensor (50), configured to measure the at least one process parameter and to generate a sensed value representing the at least one process parameter;
a movement mechanism (40), configured to move the sensor (50) inside the passage (70) to the predefined location (75);
a holding mechanism (30), configured to hold the sensor (50) at the predefined location (75), after the sensor (50) has moved to the predefined location (75);
a communication mechanism (60), configured to enable data communication to and from the sensor system (10), data communication being related to aspects including to control at least one of the movement mechanism (40), the holding mechanism (30), and/or the sensor (50).

2. The sensor system (10) of claim 1, further comprising an energy supply mechanism (15),
wherein the energy supply mechanism (15) comprises an energy storage (16) and/or an energy generator (17).

3. The sensor system (10) of claim 2,
wherein the energy generator (17) comprises a magnetic and/or electromagnetic coupler and/or energy harvesting means.

4. The sensor system (10) of any one of the preceding claims,
wherein the movement mechanism (40) comprises a propulsion mechanism, a broadened portion, a rudder, and/or a sail.

5. The sensor system (10) of any one of the preceding claims,
wherein the holding mechanism (30) comprises magnetic means, friction means, and/or Van der Waals means.

6. The sensor system (10) of any one of the preceding claims,
wherein the communication mechanism (60) comprises a transceiver (62), which is configured to communicate electromagnetically, magnetically, optically, and/or acoustically.

7. The sensor system (10) of any one of the preceding claims, wherein the movement mechanism (40) and/or the holding mechanism (30) comprises:
a body (20),
a leg (32), which is mounted on the body (20) in a moveable way, and
a foot (34), which is mounted on the leg (32),
wherein the movement mechanism (40) comprises means for spreading the leg (32), and/or
wherein the holding mechanism (30) comprises means for folding the leg (32).

8. The sensor system (10) of claim 7,
wherein spreading the leg (32) comprises means for axially turning the leg (32), thus increasing the drag coefficient of the leg (32).

9. The sensor system (10) of any one of the preceding claims,
wherein the passage (70) is partly and/or in sections open, and/or is designed as a container, a tube, a pipeline, and/or as a vasculature.

10. A method for sensing at least one process parameter and for generating a sensed value representing the at least one process parameter, by a sensor system (10), at a predefined location (75) inside a passage (70) comprising a medium (72), the method comprising the steps of:
inserting the sensor system (10) into the medium (72) of the passage (70);
guiding the sensor system (10), by means of a movement mechanism (40), to the predefined location (75);
holding the sensor system (10), by means of a holding mechanism (30), at the predefined location (75); and
measuring the at least one process parameter and generating a sensed value representing the at least one process parameter, by means of a sensor (50), at the predefined location (75).

11. The method of claim 10, comprising the further step of:
releasing the sensor system (10), by means of the holding mechanism (30).

12. The method of claim 10 or 11,
wherein holding the sensor system (10) comprises building a detachable connection between the sensor system (10) and the passage (70), and/or
keeping the structure of the passage (70) essentially unchanged.

13. The method of any one of the claims 10 to 12,
wherein at least one of inserting, guiding, and/or holding the sensor system (10), measuring the at least one process parameter and generating a sensed value representing the at least one process parameter, and/or releasing the sensor system (10) is controlled by a communication mechanism (60).

14. The method of any one of the claims 10 to 13,
wherein guiding the sensor system (10) comprises to move the sensor system (10), by means of a propulsion mechanism, and/or
wherein the medium (72) is a flowing medium, and wherein the guiding comprises a steering, by means of a rudder and/or a sail.

15. Use of a sensor system (10) according to any one of claims 1 to 9 or a method according to any one of claims 10 to 14 for measuring at least one process parameter and generating a sensed value representing the at least one process parameter inside a passage (70) at a predefined location (75).
